# EUROPÄISCHE PATENTSCHRIFT

(11) **EP 1 069 947 B1**
(45) Veröffentlichungstag und Bekanntmachung des Hinweises auf die Patenterteilung: **31.10.2001**
(21) Anmeldenummer: 99917880.9
(22) Anmeldetag: 26.03.1999
(51) Int. Cl.: B01J 23/847, B01J 23/887, B01J 23/888, B01J 23/83, C07C 5/333, C07C 15/50

(54) **KATALYSATOR ZUR DEHYDRIERUNG VON 1,1-DIPHENYLETHAN UND SUBSTITUIERTEN 1,1-DIARYLETHANEN**
CATALYST FOR DEHYDROGENATING 1,1-DIPHENYLETHANES AND SUBSTITUTED 1,1-DIARYLETHANES
CATALYSEUR POUR DESHYDROGENER DU 1,1-DIPHENYLETHANE ET DES 1,1-DIARYLETHANES SUBSTITUES

(30) Priorität: 30.03.1998 DE 19814081
(43) Veröffentlichungstag der Anmeldung: 24.01.2001
(73) Patentinhaber: BASF AKTIENGESELLSCHAFT, 67056 Ludwigshafen (DE)
(72) Erfinder: BAIER, Michael, D-68161 Mannheim (DE)
(86) Internationale Anmeldenummer: EP9902084
(87) Internationale Veröffentlichungsnummer: WO9949967

(56) Entgegenhaltungen:
- EP-A- 0 177 832
- DE-A- 2 815 812
- DE-A- 2 815 874
- GB-A- 2 280 126
- US-A- 4 365 103
- PATENT ABSTRACTS OF JAPAN vol. 14, no. 552 (C-0778), 15. November 1990 (1990-11-15) -& JP 02 215734 A (ASAHI CHEM IND CO LTD), 28. August 1990 (1990-08-28) in der Anmeldung erwähnt

## Beschreibung

Die Erfindung betrifft einern Katalysator für ein insbesondere kontinuierlich durchführbares Verfahren zur Herstellung von 1,1-Diphenylethylen ("Diphenylethylen"; DPE) durch Dehydrierung von 1,1-Diphenylethan ("Diphenylethan"; DPEA). DPE wird zur Herstellung von Copolymeren mit Styrol benötigt, die eine höhere Erweichungstemperatur als Styrol-Homopolymere aufweeisen.

Es ist bekannt, daß DPEA analog dem Verfahren zur Gewinnung von Styrol aus Ethylbenzol zu DPE dehydriert werden kann (vgl. z.B. US-Patentschrift 4,365,103). Ryabow et al., Khim.Prom. 1983, 398-400; (Übs. Sov.Chem.Ind. 83, 784 ff) erhielten DPE bei einem Verhältnis von Wasserdampf:DPEA von 8,5:1 [v/v] bei atmosphärischem Druck, 500 bis 520°C und einer Kontaktzeit von 11,6 Sek. mit einer Selektivität von 95% bei einer Ausbeute von 66%. Diese Autoren beschreiben auch, daß DPEA durch destillative Weiterverarbeitung der Rückstände der Herstellung von Ethylbenzol auf 72,6% angereichert und dann bei 550 bis 600°C, einer Raum-Zeit-Ausbeute (LHSV; liquid hourly space velocity) von 0,4 [h⁻¹] und Organisch/ Wasser-Verhältnissen von 1:4 zu DPE dehydriert werden kann (Sov.Chem.Ind. 83, 784 ff (10), 1985, 45-47). Ryabov et.al., loc. cit. beschreiben weiter, daß sie bei einem Verhältnis Dampf:Diphenylethan von 8,5:1 [v/v] bei atmosphärischem Druck und 500 bis 520°C und einer Kontaktzeit von 11,6 Sek. DPE in 66%iger Ausbeute mit einer Selektivität von 95% erhielten.

US-Patentschrift 2,450,334 beschreibt die Dehydrierung von 1,1-Diarylalkanen zu den entsprechenden Alkenen an Kupferchromit-Aluminiumoxid-Katalysatoren. Nach S.M. Aliev und Sh.S. Vezirov (Azerb. Neft. Khoz., 53(7), 1973, 35-36 und Neftekhimiya, 23(3), 1983, 323-330) kann DPE aus reinem DPEA durch Dehydrierung an einem Fe-Cr-K-Katalysator oder einem industriellen Mischoxidkatalysator in Gegenwart von Wasserdampf (Verhältnis Dampf:DPEA = 4:1 [v/v]) bei einem Umsatz von 40% mit einer Selektivität von ca. 90% hergestellt werden. Die LHSV betrug bei den Versuchen 0,4 h⁻¹; als Temperatur ist 475°C angegeben.

Nach S.H. Vezirov; Neftekhimiya, 21(1), 1981, 28-32 kann die Dehydrierung von p-Isopropyl-1,1-diphenylethan an einem Mischoxidkatalysator in Gegenwart von Dampf bei 400 bis 600°C durchgeführt werden. Bei einer LHSV von 0,4 h⁻¹, einem Verhältnis Dampf:Organisch von 10:1 [v/v] und einer Temperatur von 600°C beträgt die Ausbeute 84% bei einem Umsatz von 90% (entsprechend einer Selektivität: 93,33%).

Nach JA 01 013 048 und JA 01 013 052 kann die Dehydrierung von ethylsubstituierten 1,1-Diphenylethanen an Eisenoxid- und/oder Chromoxid-Katalysatoren bei 400 bis 650°C vorgenommen werden.

Nach JA 2215-734 kann die Umsetzung an einem Katalysator aus Feoxid (40 bis 85 Gew.-%), K₂CO₃ (10 bis 50 Gew.-%), Mo-Oxid (2 bis 6 Gew.-%), Se-Oxid (2 bis 6 Gew.-%) vorgenommen werden.; Reaktionsbedingungen: Dampf/Organisch = 10 bis 100, bevorzugt: 20 bis 100 mol/mol, LHSV = 0,05 bis 5 h⁻¹, T = 500-650°C; Umsatz = 96,7%; Ausbeute = 90,4%.

Nach der EP-A1-0 282 066 kann 1-(3-Ethylphenyl)-1-phenylethan an Styrolkatalysatoren zu 1-(3-Vinylphenyl)-1-phenylethen dehydriert werden. Die Katalysatoren können neben Eisenoxid auch Kaliumcarbonac und Oxide des Chroms, Cers, Molybdäns oder Vanadiums enthalten. Die Umsetzung wird bei 500-700°C bei einer LHSV von 0,01-10 h⁻¹ vorgenommen.

Nach der US-Patentschrift 4,365,103 können Bis-arylethane an Fe/K/Cr-Katalysatoren dehydriert werden, wie sie bei der Dehydrierung von Ethylbenzol eingesetzt werden.

Andere Katalysatoren sollen durch Zusatz verhältnismäßig hoher Cer-Mengen (EP-A-0 297 685: 10-60 Gew.-% Ce₂O₃) günstig beeinflußt werden: In den Unterlagen der DE-A-28 15 874 und DE-A-28 15 812 sind Katalysatoren beschrieben, die neben Eisen, Kalium, Molybdän ebenso wie Wolfram auch Cer und darüber hinaus auch Vanadium enthalten.

Bekannt ist weiter ein Katalysator zur Dehydrierung von Ethylbenzol zu Styrol im sog. adiabaten Verfahren. Zur Herstellung wird nach EP A 0 195 252 ein handelsübliches Eisenoxidpigment (α-FeOOH) verwendet. Neben Eisen enthält der Katalysator in einem konkreten Beispiel Oxide von Kalium, Calcium, Wolfram und Cer. Zur Herstellung wird aus Eisenoxid, K₂CO₃, CaCO₃, WO₃ und Ce₂(CO₃)₃ eine sorgfältige Mischung hergestellt und diese nach Zugabe von Wasser 3 h lang geknetet. Die so erhaltene pastose Kontaktmasse wird zu Strängen mit einem Durchmesser von 6 mm extrudiert, 1 h lang bei 80°C getrocknet und anschließend 2 h lang bei 400°C calciniert. Ein solcher Katalysator kann z.B. aus 1000 Gew.-Teilen α-FeOOH, 357 Gew.-Teilen K₂CO₃, 62 Gew.-Teilen WO₂, 111 Gew.-Teilen Ce₂(CO₃)₃ und 208 Gew.-Teilen CaCO₃ hergestellt werden. Weitere Katalysatoren, die sich insbesondere zur Dehydrierung von Ethylbenzol zu Styrol eignen, sind z.B. in US 3 904 552, DE-A1-3 442 636, EP-A1-188 999, EP-A1-177 832, US 4 467 046, US 4 749 674, DE 28 15 874, DE 28 15 812, EP-A1-297 657, EP-A1-502 510, EP-A1-297 685 und WO 94/25 154 beschrieben worden. Weitere Verweise zur allgemeinen Kenntnisstand befinden sich dort.

Zwar ist es teilweise bekannt, daß sich diese speziellen Katalysatoren zur Dehydrierung von 1,1-Diarylethanen eignen. Andererseits ist allgemein bekannt, daß Katalysatoren gegenwärtig für einen bestimmten Einsatzzweck maßgeschneidert werden müssen. Die vorstehend angegebenen Dokumente können daher zu der Auffindung neuer Katalysatoren nur wenig beitragen. Es hat sich insbesondere erwiesen, wie nachstehend noch im einzelnen gezeigt werden wird, daß die bekannten Katalysatoren zur Dehydrierung von DPEA zu DPE nicht gleich geeignet sind wie zur Dehydrierung von Ethylbenzol zu Styrol. Sie sind insbesondere hinsichtlich der Selektivität der Dehydrierung und der Aktivität bei Temperaturen von 530-570°C verbesserungsbedürftig.

Es besteht daher Bedarf für ein Verfahren zur Herstellung von 1,1-Diphenylethylen und dessen technischen Äquivalenten, d.h. substituierten 1,1-Diarylethenen, das dem bekannten Verfahren zur Herstellung von Styrol aus Ethylbenzol nachgebildet ist.

Aufgabe der Erfindung ist die Schaffung von Katalysatoren, die sich besonders zur Dehydrierung von DPEA zu DPE und deren technischen Äquivalenten auch bei vergleichsweise niedrigen Temperaturen eignen.

Es wurde dementsprechend gefunden, daß zur Dehydrierung von 1,1-Diarylethanen zu den entsprechenden 1,1-Diarylethenen, insbesondere zur Dehydrierung von 1,1-Diphenylethan zu 1,1-Diphenylethen besonders geeignete Katalysatoren folgende Zusammensetzung aufweisen sollten:
50-90 Gew.-% Eisen, berechnet als Fe₂O₃;
5-40 Gew.-% Kalium, berechnet als K₂O;
0,01-30 Gew.-%, insbesondere 3-12 Gew.-% Cer, berechnet als Ce₂O₃;
0,01-10 Gew.-%, insbesondere 1-5 Gew.-% Magnesium,
   berechnet als MgO;
0,01-10 Gew.-%, insbesondere 0,5-2,5 Gew.-% Vanadium,
   berechnet als V₂O₅;
0,01-10 Gew.-%, insbesondere 1-5 Gew.-% Wolfram und/oder Molybdän, berechnet als WO₃ bzw. MoO₃.

Ein besonders vorteilhafter Katalysator enthält außerdem bis zu etwa 5 Gew.-% Aluminium, berechnet als Al₂O₃. Weiterhin vorteilhaft sind Katalysatoren, die zusätzlich bis zu etwa 10 Gew.-% Caicium enthalten, berechnet als CaO.

Dementsprechend enthält ein besonders vorteilhafter Katalysator: 50 bis 90 Gew.% Eisen; 5 bis 40 Gew.-% Kalium; 1 bis 5 Gew.-% Magnesium; 3 bis 12 Gew.-% Cer; 0,5 bis 3 Gew.-% Vanadium; 1 bis 5 Gew.-% Molybdän oder 1 bis 5 Gew.-% Wolfram oder 1 bis 5 Gew.-% einer Mischung von Molybdän und Wolfram, 0,1 bis 5 Gew.-% Aluminium sowie 0,1 bis 10 Gew.-% Calcium, berechnet jeweils als Oxide.

Alle Katalysatoren enthalten Eisen, Kalium, Cer, Wolfram und/oder Molybdän. Wesentliches Merkmal der Katalysatoren ist die zusätzliche Verwendung von Vanadium und Magnesium. Vanadium verbessert gleichzeitig Aktivität und Selektivität der Katalysatoren. In gleicher Richtung wirkt die zusätzliche Verwendung von Magnesium. Die Kombination dieser beiden Promotoren wirkt sich insbesondere im Temperaturbereich zwischen 500 und 570°C aktivitäts- und selektivitäts-steigernd aus.

Aluminium und/oder Calcium erhöhen die Langzeitstabilität der Katalysatoren und gehören deshalb zur bevorzugten Rezeptur für die erfindungsgemäßen Katalysatoren. Die Promotierung mit Aluminium und/oder Calcium ist aber nicht unbedingt notwendig.

Zur Herstellung der erfindungsgemäßen Katalysatoren ist im einzelnen das Folgende zu sagen:

### Einsatzstoffe

Eisen, Kalium sowie gegebenenfalls Promotoren werden in Form von Oxiden oder von Substanzen, die sich beim Erhitzen in Oxide umwandeln, miteinander gemischt.

Als Eisenverbindung wird bevorzugt Fe₂O₃, FeOOH, Fe₃O₄ oder eine andere künstliche oder natürlich vorkommende oxidische Eisenverbindung verwendet.

Als Kaliumverbindung wird bevorzugt Kaliumcarbonat, Kaliumhydroxid oder eine andere in der Wärme zersetzbare Kaliumverbindung wie Kaliumoxalat verwendet. Man kann auch eine Kaliumverbindung verwenden, die den vorgesehenen Promotor (d.h. als entsprechendes Anion oder als Doppelsalz) enthält.

Als Vanadiumverbindung werden bevorzugt V₂O₅, Ammoniumvanadat oder Alkalimetallvanadate verwendet.

Als Magnesiumverbindung wird bevorzugt Mg(OH)₂, MgO, MgCO₃ oder Magnesiumhydroxycarbonat eingesetzt.

Als Cerverbindung wird bevorzugt Ce₂O₃, Ceroxalat oder Cercarbonat verwendet.

Als Molybdänverbindung wird bevorzugt MoO₃, H₂MoO₄ oder Ammoniummolybdat verwendet.

Als Wolframverbindung wird bevorzugt WO₃, H₂WO₄ oder Ammoniumwolframit verwendet.

Als Aluminiumverbindung wird bevorzugt AlOOH oder Al₂O₃ verwendet.

Als Calciumverbindung wird bevorzugt CaO, CaCO₃ oder Ca(OH)₂ verwendet.

### Mischen der Einsatzstoffe und Herstellung einer verformbaren Masse

Wichtig ist eine innige Durchmischung der Einsatzstoffe. Diese kann einfach durch trockene Mischung oder durch Suspendierung der Einsatzstoffe in Wasser und Sprühtrocknung der gebildeten Maische hergestellt werden. Bei allen Mischvorgängen ist es von Vorteil alle Bestandteile mit Ausnahme des Eisenoxids in möglichst feingepulverter Form vorzulegen. Aus den Mischungen erhält man nach Wasserzugabe durch Kneten oder Kollern eine verformbare Masse. Die Verformung zu Tabletten kann dagegen mit einer trockenen Mischung der Bestandteile durchgeführt werden.

Die Mischung der Einsatzstoffe und die Herstellung einer verformbaren Masse kann auch in einer einzigen Apparatur (z.B. in einem Koller) in einem Schritt durchgeführt werden.

### Herstellung von Formkörpern

Formkörper lassen sich außer durch Extrusion oder Verstrangung auch durch Tablettierung des trockenen Sprühpulvers oder eines Gemisches in einer Tablettenpresse herstellen. In diesem Fall kann es von Nutzen sein, Tablettierungshilfsmittel (z.B. Graphit, verschiedene Stearate) zum Tablettiergut zuzusetzen. Als Formkörper kommen Stränge unterschiedlicher Geometrie in Frage, bevorzugt Vollstränge mit einem Durchmesser von z.B. 3-6 mm. Ebenso geeignet sind z.B. Hohlstränge oder sog. Rib- oder Sternstränge (d.h. Stränge mit äußeren Längsrillen oder -rippen bzw. sternförmigem Querschnitt) oder Ringtabletten mit Innenloch. Die Verformbarkeit kann mit Hilfsmitteln wie Stearaten, Walocel, Stärke oder ähnlichem beeinflußt werden. Nach der Verformung werden die Stränge üblicherweise durch geeignete Einrichtungen wie z.B. Messer abgelängt.

### Trocknung

Die Trocknung kann kontinuierlich oder diskontinuierlich durchgeführt werden. Für die kontinuierliche Trocknung kommen Bandtrockner, für die diskontinuierliche Trocknung Hordenöfen in Frage. Im technischen Maßstab sind die für großtechnische Trockenverfahren geeigneten Anlagen verwendbar wie Bandtrockner oder Hordenöfen. Übliche Trocknungstemperaturen sind 80-140°C. Höhere Trocknungstemperaturen können aufgrund einer zu hohen Trocknungsgeschwindigkeit zu einem unerwünschten Aufplatzen der Formkörper führen. Niedrigere Trocknungstemperaturen sind möglich, verlängern aber die Trocknungszeit.

### Temperung; Calcinierung

Nach dem Trocknen werden die Formkörper zuerst für ca. 2 h auf 250 bis 350°C ("Temperung"), dann für 1 bis 2 h auf 600 bis 1000°C erhitzt ("Calcinierung"). Bei der Herstellung in technischem Maßstab kann das Tempern und Calcinieren in einem Arbeitsgang z.B. in einem Drehrohr mit unterschiedlichen Heizzonen durchgeführt werden. Die Temperatur wird dann schrittweise von z.B. 250 auf 800 bis 900°C erhöht. Nach dem Austritt aus dem Drehrohr läßt man die Formkörper abkühlen. Bruchstücke und Feinstaub werden durch Siebung abgetrennt und verworfen.

Die Erfindung umfaßt auch das Verfahren zur Dehydrierung von 1,1-Diarylethanen zu 1,1-Diarylethenen, insbesondere von 1,1-Diphenylethan zu 1,1-Diphenylethen in Gegenwart von überhitztem Wasserdampf unter Verwendung eines erfindungsgemäßen Katalysators.

### Beispiele und Vergleichsversuche

Variiert wurden Zusammensetzung und Calciniertemperatur. Sämtliche Herstellungsschritte wurden bei allen Katalysatoren in der gleichen Art und Weise durchgeführt, die anhand des Beispiels 1 erläutert ist:

900 g α-Fe₂O₃, das in Form von Nadeln mit einer Länge von 0,4 µm und einem Verhältnis "Länge/Breite" von ca. 5 vorlag, wurden unter Rühren zu einer Suspension gegeben, die außerdem 215 g K₂CO₃, 61,4 g wasserhaltiges Cercarbonat (Zusammensetzung der Formel Ce₂(CO₃)₃·xH₂O mit einem Cergehalt von 40 Gew.-%), 18 g WO₃, 14 g V₂O₅, 5 g AlOOH und 56,1 g basisches Magnesiumcarbonat (Zusammensetzung der Formel 4MgCO₃·Mg(OH)₂·4H₂O entsprechend 48 g MgCO₃) in 2000 ml Wasser enthielt. Die Suspension wurde sprühgetrocknet. Das Sprühpulver wurde in einem Kneter unter Zugabe von ca. 120 ml Wasser innerhalb von 30 Minuten zu einer pastosen Masse verarbeitet. Diese wurde in einer Strangpresse zu zylindrischen Vollsträngen von 3 mm Durchmesser geformt, in ca. 1 cm lange Stücke geschnitten, in einem Umluftofen bei 100°C ca. 2 Stunden getrocknet und in einem Calcinierofen erst 2 Stunden bei 300°C getempert und dann bei 720°C calciniert. Der Katalysator wurde in der Laboranlage als Splitt eingesetzt. Dazu wurden die Stränge in einer Rotormühle vermahlen und das Mahlgut gesiebt. Für die Test wurde Splitt der Siebfraktion 0,5 bis 0,7 mm verwendet.

Da es grundsätzlich bekannt war, daß sich Katalysatoren für die Dehydrierung von Ethylbenzol auch für die Dehydrierung von 1,1-Diphenylethan eignen (vgl. oben), wurde außerdem ein Katalysator hergestellt, (V1), wie er von S.M. Aliev et.al. (siehe oben!) für die Dehydrierung von 1,1-Diarylethanen und von 1,1-Diphenylethan bereits früher eingesetzt worden war. Dieser als Vergleichskatalysator V1 eingesetzte Katalysator entspricht einem handelsüblichen, früher für die Dehydrierung von Ethylbenzol verwendeten Katalysator auf der Grundlage Eisen, Kalium und Chrom.

Man erkennt, daß die erfindungsgemäßen Katalysatoren deutliche Vorteile bei der Selektivität gegenüber dem von diesen Autoren verwendeten Katalysator haben. Die erfindungsgemäßen Katalysatoren erzielen bei vergleichbaren Bedingungen, d.h. vergleichbarem Umsatz deutlich höhere Selektivitäten als der Vergleichskatalysator V1.

Da andererseits solche Eisen-Kalium-Chrom-Katalysatoren bei der Dehydrierung von Ethylbenzol nicht mehr als die derzeit besten gelten, wurde als weiterer Vergleichskatalysator (V2) ein Katalysator eingesetzt, der durch den Zusatz weiterer Promotoren (hier Calcium, Wolfram und Cer) bei der Dehydrierung von Ethylbenzol zwar eine geringere Aktivität, dafür aber eine deutlich höhere Selektivität erzielt.

Vergleicht man die erfindungsgemäßen Katalysatoren mit V2, so erkennt man, daß deren Aktivität bei 550°C deutlich höher als die von V2 ist. Auch die Selektivität ist signifikant höher.

Es wurden außerdem eine Reihe von Katalysatoren (V3-V5) hergestellt und zur Dehydrierung von DPEA eingesetzt, die in der Zusammensetzung verschiedenen gegenwärtig handelsüblichen Katalysatoren entsprechen, die für die Dehydrierung von Ethylbenzol als besonders geeignet beschrieben sind.

Darunter waren Katalysatoren auf Basis Fe₂O₃, die als Promotoren Cer, Calcium und Wolfram oder Molybdän und Cer enthielten. Diese hatten alle wie der Katalysator V2 deutliche Nachteile gegenüber den erfindungsgemäßen Katalysatoren. Sie hatten bei 550°C eine deutlich schlechtere Aktivität und/ oder eine schlechtere Selektivität (vgl. Tabelle 2).

Überraschenderweise werden die Aktivität und die Selektivität der erfindungsgemäßen Katalysatoren für die Dehydrierung von 1,1-Diphenylethan durch den Zusatz des Promotors Vanadium erhöht. Hier liegt ein ganz wesentlicher Unterschied zur Dehydrierung von Ethylbenzol. Vanadium ist dort ausschließlich ein Selektivpromotor, der zu einer deutlichen Aktivitätsverminderung führt.

### Versuche zur Dehydrierung

In einem 6 mm weiten, in einem Salzbad beheizbaren Rohrreaktor wurden jeweils 11,5 ml Katalysatorsplitt der Siebfraktion 0,5 bis 0,7 mm eingebaut. Die Versuche wurden in einem Salzbad (Carbonatschmelze) bei einer Badtemperatur von 610°C begonnen. Zunächst wurde durch Eichung einer Membranpumpe ein konstanter Volumenstrom von 18,3 ml flüssigem Wasser pro Stunde eingestellt. Nach Erreichen der Konstanz wurde diese Fördermenge durch Überleitung über eine heiße Steatitschüttung kontinuierlich verdampft, auf 610°C aufgeheizt und über die Katalysatorschüttung geleitet. Nach 1 Stunde wurde ein ebensolcher Volumenstrom von 25 ml DPEA pro Stunde (bezogen auf die Flüssigkeit) auf dieselbe Weise verdampft, mit dem Wasserdampf gemischt und die Mischung anstelle des reinen Wasserdampfs über den Katalysator geleitet (LHSV = 2,17 h⁻¹). Wenn nach ca. 10 Tagen der Katalysator ein stabiles Umsatz- und Selektivitätsniveau erreicht hatte, wurden Zusammensetzung der flüssigen Phase und Abgasmenge und -zusammensetzung bilanziert. Danach wurden die Versuche bei 550°C fortgesetzt und nach 3 Tagen eine weitere Bilanzierung vorgenommen.

Tabelle 1 gibt Zusammensetzung, Calciniertemperatur und Leistung der erfindungsgemäßen Katalysatoren 1 bis 5 und der Vergleichskatalysatoren V1 bis V2 wieder; Mengen jeweils in dg.

Tabelle 2 gibt Zusammensetzung, Calciniertemperatur und Leistung des erfindungsgemäßen Katalysators gem. Beispiel 1 und der Vergleichskatalysatoren V2-V5 wieder; Mengen jeweils in dg.

**Tabelle 1**

| Beispiele/Vergl. | 1 | 2 | 3 | 4 | 5 | V1 | V2 |
|---|---|---|---|---|---|---|---|
| α-Fe₂O₃ | 90 | 90 | 90 | 90 | - | 90 | 90 |
| α-FeOOH | - | - | - | - | 100 | - | - |
| K₂CO₃ | 21,5 | 21,5 | 21,5 | 21,5 | 21,5 | 21,5 | 19,0 |
| CrO₃ | - | - | - | - | - | 3,0 | - |
| Ce₂(CO₃)3·xH₂O | 6,14 | 6,14 | 6,14 | 6,14 | 6,14 | - | 11,4 |
| V₂O₅ | 1,4 | 1,4 | 1,4 | 1,4 | 1,4 | - | - |
| 4MgCO₃·Mg(OH)₂·4H₂O | 5,6 | 5,6 | 5,6 | 5,6 | 5,6 | - | - |
| WO₃ | 1,8 | - | 1,8 | 1,8 | 1,8 | - | 4,4 |
| MoO₃ | - | 1,2 | - | - | - | - | - |
| AlOOH | 0,5 | 0,5 | 0,5 | 0,5 | 0,5 | - | - |
| CaCO₃ | - | - | - | 1,8 | - | - | 4,2 |
| T_{calc}[°C] | 720 | 720 | 850 | 720 | 720 | 750 | 850 |
| U_{610°C} [%] | 83 | 84 | 81 | 78 | 85 | 85 | 80 |
| S_{610°C} [%] | 98 | 98,5 | 99,0 | 99,3 | 98,2 | 91 | 97,0 |
| U_{550°C} [%] | 49 | 50 | 46 | 42 | 51 | 48 | 32 |
| S_{550°C} [%] | 99,9 | 99,8 | 99,9 | 99,9 | 99,5 | 95 | 98,5 |

**Tabelle 2**

| Beispiel/Vergleich | 1 | V2 | V3 | V4 | V5 |
|---|---|---|---|---|---|
| α-Fe₂O₃ | 90 | 90 | 90 | 90 | 90 |
| K₂CO₃ | 21,5 | 19,0 | 21,5 | 21,5 | 21,5 |
| Ce₂(CO₃)₃·xH₂O | 6,14 | 11,4 | 6,14 | 6,14 | 6,14 |
| V₂O₅ | 1,4 | - | - | 1,4 | 1,4 |
| 4MgCO₃·Mg(OH)₂·4H₂O | 5,6 | - | 5,6 | - | - |
| WO₃ | 1,8 | 4,4 | 1,8 | 1,8 | 1,8 |
| AlOOH | 0,5 | - | 0,5 | 0,5 | 0,5 |
| CaCO₃ | - | 4,2 | - | - | - |
| T_{calc}[°C] | 720 | 850 | 720 | 720 | 850 |
| U_{610°C} | 83 | 80 | 81 | 81 | 78 |
| [%] | | | | | |
| S_{610°C} | 98 | 97,0 | 94,5 | 97,0 | 97,5 |
| [%] | | | | | |
| U_{550°C} | 49 | 32 | 47 | 44 | 42 |
| [%] | | | | | |
| S_{550°C} | 99,9 | 98,5 | 97,4 | 99,5 | 99,7 |
| [%] | | | | | |

## Patentansprüche

1. Katalysator, enthaltend
50 bis 90 Gew.-% Eisen, berechnet als Fe₂O₃;
5 bis 40 Gew.-% Kalium, berechnet als K₂O;
0,01 bis 30 Gew.-% Cer, berechnet als Ce₂O₃;
0,01 bis 10 Gew.-% Magnesium, berechnet als MgO;
0,01 bis 10 Gew.-% Vanadium, berechnet als V₂O₅ sowie
0,01 bis 10 Gew.-% Wolfram und/oder Molybdän,
berechnet als WO₃ bzw. MoO₃.

2. Katalysator nach Anspruch 1, enthaltend 0,01 bis 5 Gew.-% Aluminium, berechnet als Al₂O₃.

3. Katalysator nach Anspruch 1, enthaltend 0,01 bis 10 Gew.-% Calcium, berechnet als CaO.

4. Katalysator nach Anspruch 1, enthaltend 1 bis 5 Gew.-% Magnesium, 3 bis 12 Gew.-% Cer, 0,5 bis 2,5 Gew.-% Vanadium und 1 bis 5 Gew.-% Molybdän oder Wolfram oder 1 bis 5 Gew.-% einer Mischung von Molybdän und Wolfram, jeweils berechnet als Oxid.

5. Verfahren zur nicht-oxidativen Dehydrierung von 1,1-Diarylethanen zu 1,1-Diarylethenen in Gegenwart von überhitztem Wasserdampf, **dadurch gekennzeichnet, daß** man einen Katalysator nach einem der Ansprüche 1 bis 4 verwendet.

6. Verfahren nach Anspruch 5 zur Dehydrierung von 1,1-Diphenylethan zu 1,1-Diphenylethen, **dadurch gekennzeichnet, daß** man einen Katalysator nach einem der Ansprüche 1 bis 4 verwendet.

## Claims

1. A catalyst containing
from 50 to 90% by weight, calculated as Fe₂O₃, of iron,
from 5 to 40% by weight, calculated as K₂O, of potassium,
from 0,01 to 30% by weight, calculated as Ce₂O₃, of cerium,
from 0,01 to 10% by weight, calculated as MgO, of magnesium,
from 0,01 to 10% by weight, calculated as V₂O₅, of vanadium, and
from 0,01 to 10% by weight, calculated as WO₃ or MoO₃, of tungsten or molybdenum.

2. A catalyst as claimed in claim 1, containing from 0.01 to 5% by weight, calculated as Al₂O₃, of aluminum.

3. A catalyst as claimed in claim 1, containing from 0.01 to 10% by weight, calculated as CaO, of calcium.

4. A catalyst as claimed in claim 1, containing from 1 to 5% by weight of magnesium, from 3 to 12% by weight of cerium, from 0.5 to 2.5% by weight of vanadium and from 1 to 5% by weight of molybdenum, or tungsten or from 1 to 5% by weight of a mixture of molybdenum and tungsten, calculated in each case as the oxide.

5. A process for the nonoxidative dehydrogenation of 1,1-diarylethanes to 1,1-diarylethenes in the presence of superheated steam, wherein a catalyst as claimed in any of claims 1 to 4 is used.

6. A process as claimed in claim 5 for the dehydrogenation of 1,1-diphenylethane to 1,1-diphenylethene, wherein a catalyst as claimed in any of claims 1 to 4 is used.

## Revendications

1. Catalyseur contenant :
de 50 à 90 % en poids de fer, calculés en tant que Fe₂O₃ ;
de 5 à 40 % en poids de potassium, calculés en tant que K₂O ;
de 0,01 à 30 % en poids de cérium, calculés en tant que Ce₂O₃ ;
de 0,01 à 10 % en poids de magnésium, calculés en tant que MgO ;
de 0,01 à 10 % en poids de vanadium, calculés en tant que V₂O₅ ; ainsi que
de 0,01 à 10 % en poids de tungstène et/ou de molybdène
calculés en tant que WO₃ ou MoO₃.

2. Catalyseur selon la revendication 1, contenant de 0,01 à 5 % en poids d'aluminium, calculés en tant qu'Al₂O₃.

3. Catalyseur selon la revendication 1, contenant de 0,01 à 10 % en poids de calcium, calculés en tant que CaO.

4. Catalyseur selon la revendication 1, contenant de 1 à 5 % en poids de magnésium, de 3 à 12 % en poids de cérium, de 0,5 à 2,5 % en poids de vanadium et de 1 à 5 % en poids de molybdène ou de tungstène, ou de 1 à 5 % en poids d'un mélange de molybdène et de tungstène, chaque fois calculés sous la forme de l'oxyde.

5. Procédé de déshydrogénation non oxydante de 1,1-diaryléthanes en 1,1-diaryléthènes, en présence de vapeur d'eau surchauffée, **caractérisé en ce que** l'on utilise un catalyseur selon l'une des revendications 1 à 4.

6. Procédé selon la revendication 5, pour la déshydrogénation de 1,1-diphényléthane en 1,1-diphényléthène, **caractérisé en ce que** l'on utilise comme catalyseur un catalyseur selon l'une des revendications 1 à 4.
